(19) Europäisches Patentamt
European Patent Office
Office européen des brevets

(11) **EP 4 751 703 A1**

(12) **DEMANDE DE BREVET EUROPEEN**

(43) Date de publication:
**03.06.2026 Bulletin 2026/23**

(21) Numéro de dépôt: **25218356.1**

(22) Date de dépôt: **25.11.2025**

(51) Classification Internationale des Brevets (IPC):
*A61K 8/06* (2006.01)    *A61K 8/26* (2006.01)
*A61K 8/92* (2006.01)    *A61Q 19/00* (2006.01)
*A61Q 19/08* (2006.01)

(52) Classification Coopérative des Brevets (CPC):
**A61K 8/922; A61K 8/062; A61K 8/26; A61Q 19/00;
A61Q 19/08**

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**GE KH LA MA MD TN**

(30) Priorité: **28.11.2024 FR 2413151**

(71) Demandeur: **Ephyla**
**56190 Arzal (FR)**

(72) Inventeur: **BOURGETEAU, Vincent**
**56130 FEREL (FR)**

(74) Mandataire: **Eveillard, Sophie**
**Apropis**
**44, rue César**
**56100 Lorient (FR)**

(54) **UTILISATION COSMÉTIQUE D'UNE COMPOSITION COMPRENANT UNE ÉMULSION DE PICKERING H/E CONTENANT UN PHYLLOSILICATE ORGANOMODIFIÉ EN TANT QU AGENT NEUROCOSMÉTIQUE**

(57) Utilisation cosmétique, non thérapeutique, par voie topique d'une composition en tant qu'agent neurocosmétique, ladite composition comprenant une émulsion de Pickering H/E contenant une phase aqueuse, une phase huileuse végétale et un phyllosilicate organomodifié.

**Description**

[La présente invention se rapporte au domaine de la neurocosmétique et plus particulièrement à l'utilisation d'une composition comprenant une émulsion de Pickering H/E particulière en tant qu'agent neurocosmétique.

La peau est la principale barrière de protection du corps humain à l'égard des agressions extérieures telles que la pollution atmosphérique, les variations climatiques et le rayonnement UV.

La peau est constituée de trois couches : l'épiderme, le derme et l'hypoderme. L'épiderme, qui est la couche superficielle, est plus particulièrement concerné par les interactions avec le milieu extérieur. L'épiderme est recouvert par un film hydrolipidique et est composé de quatre couches : la couche cornée, la couche granuleuse, la couche épineuse et la couche basale. L'épiderme est normalement constitué de quatre types de cellules : les kératinocytes qui représentent 80% de l'ensemble de ses cellules, les 20% restant étant composés des mélanocytes, des cellules de Langerhans ou cellules immunocompétentes et des cellules de Merkel, ces trois groupes de cellules étant dispersées entre les kératinocytes.

Plus particulièrement les disques de Merkel sont des récepteurs superficiels situés à la base de l'épiderme et composés de la terminaison d'une ramification (en forme de disque) d'une fibre myélinisée apposée à une cellule de Merkel avec laquelle elle établit des contacts synaptiques. Les zones riches en disques de Merkel peuvent former des dômes tactiles qui répondent à des pressions localisées, la réponse au stimulus étant phasico-tonique à adaptation lente.

Récemment, des recherches sur les cellules de Merkel se sont focalisées sur leur fonction dans la mécanosensation, en particulier le toucher léger (« light -touch » en langue anglaise), en raison de leur rôle primordial dans les tâches sensorielles et les interactions sociales.

En particulier, il a été été mis en évidence que les cellules de Merkel utilisent la sérotonine pour transmettre les stimulations tactiles aux terminaisons des nerfs A$\beta$-afférents avec lesquels elles sont en contact et que les stimuli tactiles activent des canneaux Piezo 2 pour transduire les stimuli mécaniques en stimuli électriques conduisant à générer des impulsions sur les nerfs A$\beta$-afférents (1).

Ces recherches ont mis en évidence l'importance de la sérotonine dans la neurotransmission des informations provenant des cellules cutanées de l'épiderme.

La neurocosmétique a été définie par le Professeur Misery en 1996, lors de ses travaux sur les relations entre la peau et le cerveau, comme visant les produits cosmétiques aptes à intervenir sur les cellules cutanées en modulant le système neuro-immuno-cutané. En 2000, le Professeur Misery décrivait les produits neurocosmétiques comme des produits appliqués sur la peau mais non absorbés et qui manifestent leur activité sur le système nerveux cutanés ou leurs effets généraux sur les médiateurs de la peau.

Depuis, la cosmétologie, c'est-à-dire la science qui étudie les mécanismes d'action des cosmétiques, leurs effets biologiques sur les humains ainsi que la façon de les utiliser, s'intéresse au développement de nouveaux ingrédients neurocosmétiques aptes à améliorer les interactions entre la peau et le système nerveux. En particulier sont recherchés des produits permettant de restaurer l'équilibre normal de la peau et plus généralement des produits permettant un retour dynamique à un équilibre physiologique corrélé à un effet de « bien être » cutané *via* la libération de neuro médiateurs induite par un stimulus physique chimique ou émotionnel (2).

Les ingrédients neurocosmétiques peuvent agir selon différents mécanismes par exemple directement sur les terminaisons des fibres nerveuses cutanées, en tant que modulateurs de la libération des neurotransmetteurs, notamment par effet « caresse » à savoir une très légère pression mécanique sur les mécano-récepteurs qui produisent ces neuromédiateurs pour transmettre l'information « caresse » au cerveau. Ils peuvent aussi moduler les fonctions des cellules non nerveuses en agissant comme molécules agonistes/antagonistes des récepteurs des neuropeptides ou comme modulateurs des effets des neurotransmetteurs.

En outre des récepteurs spécifiques de ces neuromédiateurs ainsi que les enzymes permettant de les dégrader, sont exprimées par les cellules cutanées, la liaison des neurotransmetteurs aux récepteurs spécifiques induit la modulation des propriétés des cellules et des fonctions de la peau. Par ces liens étroits, le système nerveux participe activement et significativement au maintien de l'équilibre cutané et à l'homéostasie physiologique. Or la sérotonine produite dans les cellules de Merkel présente des propriétés de neurotransmetteurs et est capable de transmettre au cerveau un message positif provenant de la peau (exemple « sensation de caresse ») et d'exercer un effet bénéfique (de type neuro-protecteur) sur les neurones sensoriels présents dans la peau.

Parmi les produits cosmétiques, qui regroupent les produits pour le maquillage, pour l'hygiène et pour le soin de la peau, cette dernière catégorie est plus particulièrement visée par la neurocosmétique notamment pour les effets suivants : anti -âge, anti-rides, tonifiant, effet apaisant des peaux qui ont subit un stress, et action eudermique c'est-à-dire les produits qui provoquent une sensation de bien être lorsqu'ils sont appliqués sur la peau.

A titre d'exemple d'ingredient neurocosmétique, on peut citer le menthol et ses dérivés qui agissent sur la peau pour la rafraichir ou la réchauffer en fonction de la formulation utilisée, de la quantité et de la zone d'application.

Les ingrédients neurocosmétiques ont été développés suite aux observations mentionnées plus haut selon lesquelles les neurones sensoriels, tout comme les cellules de l'épiderme, secrètent de multiples substances, les neuromédiateurs, qui nourrissent un dialogue permanent entre la peau et le système nerveux.

On connait déjà l'actif DEFENSIL®-SOFT commercialisé par la société Rahn qui contient un extrait de *Albatrellus confluens* et qui agit comme neuro-apaisant en prolongeant la zone de bien-être de la peau stressée et irritée, cet actif est décrit comme empêchant la sensibilisation du récepteur TRPV1 en bloquant les récepteurs de la sérotonine.

Le problème posé à l'origine de la présente invention était de proposer de nouveaux produits neurocosmétiques, capables de stimuler les neurones sensitifs cutanés afin de conférer à la peau un effet bien être, réconfortant.

## Résumé de l'invention

L'inventeur a montré qu'une solution au problème technique de proposer de nouveaux produits cosmétiques présentant des propriétés neurocosmétiques pouvait être apportée au moyen d'une composition comprenant une émulsion de Pickering particulière.

Selon un premier aspect, la présente invention vise l'utilisation cosmétique, non thérapeutique, par voie topique d'une composition en tant qu'agent neurocosmétique, ladite composition comprenant une émulsion de Pickering H/E contenant une phase aqueuse, une phase huileuse végétale et un phyllosilicate organomodifié.

Selon un deuxième aspect, la présente invention vise l'utilisation cosmétique, non thérapeutique, par voie topique de ladite composition pour apporter à la peau un effet réconfortant, une sensation de bien-être.

Selon un troisième aspect, la présente invention vise l'utilisation cosmétique, non thérapeutique, par voie topique de ladite composition pour améliorer l'hydratation de la peau et/ou améliorer l'éclat de la peau.

Selon un quatrième aspect, la présente invention vise l'utilisation cosmétique, non thérapeutique, par voie topique de ladite composition pour conférer à la peau un aspect plus lisse, plus clair et/ou plus uniforme.

Selon un cinquième aspect, la présente invention vise l'utilisation cosmétique, non thérapeutique, par voie topique de ladite composition pour son effet anti-âge, antiride et/ou anti-tâche.

Selon un sixième aspect, la présente invention vise un procédé de traitement cosmétique, non thérapeutique de la peau, comprenant au moins une étape d'application sur la peau d'une composition neurocosmétique

comprenant une émulsion de Pickering H/E contenant une phase aqueuse, une phase huileuse végétale et un phyllosilicate organomodifié.

Les compositions cosmétiques utilisées conformément à l'invention procurent un effet caresse durable, réconfortant et apportent une sensation de bien-être. Elles permettent également d'améliorer l'hydratation de la peau et de maintenir ou restaurer l'éclat du teint de la peau, ainsi que d'équilibrer la peau en surface en paliant spécifiquement à la sécheresse cutanée des zones les plus sèches et en n'ajoutant pas de corps gras sur les zones grasses. Cet effet équilibrant permet à la peau de limiter ses contrastes et confère donc un aspect plus uniforme et plus lisse. En outre, la publication (3) atteste du rôle de la sérotonine pour son action anti-âge de la peau.

Les compositions cosmétiques utilisées selon l'invention sont en outre stables, non irritantes, non toxiques, non allergisantes pour la peau.

D'autres aspects, avantages, propriétés de la présente invention sont présentés dans la description et les exemples qui suivent.

## Description détaillée

### Définitions

Dans le présent texte, sauf indications contraires spécifiques, les pourcentages sont exprimés en poids d'une composition de référence.

Dans le présent texte, les intervalles sont définis de façon abrégée afin d'éviter de décrire chacune des valeurs et toutes les valeurs de cet intervalle, cependant toute valeur appropriée dans l'intervalle peut être choisie comme la valeur supérieure, la valeur inférieure ou les valeurs terminales de l'intervalle. Par exemple, un intervalle de 0,1 à 1,0 représente les valeurs terminales de 0,1 et 1,0, ainsi que les valeurs intermédiaires de 0,2 ; 0,3 ; 0,4 ; 0,5 ; 0,6 ; 0,7 ; 0,8 ; 0,9 et tous les intervalles intermédiaires compris dans 0,1 à 1,0, tels que 0,2 à 0,5 ; 0,2 à 0,8 ; 0,7 à 1,0... Sauf indications contraires, un intervalle défini comme étant « compris entre la valeur A et la valeur B » inclut les valeurs A et B et est donc équivalent à un intervalle « allant de la valeur A à la valeur B » , l'expression « au moins » comprend la valeur énoncée après, par exemple, «au moins 5 % » doit être compris comme comprenant également « 5 % », l'expression « un maximum de » comprend la valeur énoncée après, par exemple, « un maximum de 5 % » doit être compris comme comprenant également « 5 % ».

En outre, dans le présent texte, les valeurs mesurables, telles qu'une quantité, doivent être comprises comme comprenant les écarts types qui peuvent être facilement déterminés par l'homme du métier dans le domaine technique de référence. De préférence, ces valeurs sont destinées à comprendre des variations de $\pm$ 5 %.

Par « peau » on entend l'épiderme du visage ou du corps ou du cuir chevelu.

Les compositions selon la présente invention sont des compositions cosmétiques, non thérapeutiques. Les compositions cosmétiques sont destinées à être appliquées sur les peaux saines pour traiter l'épiderme. Elles sont conformes au règlement CE 1223/2009.

Par « neurocosmétique », on entend un agent qui, lorsqu'il est appliqué sur la peau, présente une activité sur le système nerveux cutané ou des effets généraux sur les médiateurs de la peau.

En particulier, les agents neurocosmétiques ne sont généralement pas absorbés par la peau, dans le cadre de l'invention ils agissent plutôt en stimulant les neurones cutanés par une légère pression.

Au sens de la présente invention, le médiateur de la peau impliqué est la sérotonine, également appelée 5-hydroxytryptamine (5-HT) qui est un neurotransmetteur connu pour faciliter la communication des neurones du système nerveux central.

Sans vouloir être lié à une théorie quelconque, il apparait que l'application sur la peau de la composition comprenant une émulsion de Pickering particulière permet de stimuler les neurones cutanés et de conférer un effet bien-être. Cet effet conféré par la structure galénique de la composition est biomimétique du réconfort que procure une légère caresse ou encore l'action de souffler sur une égratignure, cet effet peut encore être nommé d'« effet caresse ».

Cet effet est particulièrement présent lorsque la composition est appliquée par pulvérisation. Les fines gouttelettes déposées restent sur la surface de la peau imitant ainsi les cornéocytes. La légère pression exercée sur la peau permet de stimuler les mécanorécepteurs et de libérer des neuropeptides qui envoient au cerveau des signaux similaires à ceux d'une caresse légère et agréable. Ce signal est apte à endormir tout message d'inconfort envoyé au cerveau pour le remplacer par un message de bien être.

**Emulsions de Pickering**

Les émulsions de Pickering sont des émulsions stabilisées par des particules solides. Lors de la préparation de l'émulsion, lesdites particules solides se positionnent à l'interface entre la phase aqueuse et la phase huileuse.

Les émulsions de Pickering utilisées selon la présente invention sont des émulsions huile dans eau, c'est-à-dire H/E, stabilisées par une argile particulière qui est un phyllosilicate naturel modifié.

Avantageusement, la phase aqueuse de l'émulsion de Pickering est présente en une quantité allant de 43% à 93%, de préférence 55% à 70% et de manière préférée 58% à 65% en poids par rapport au poids total de la composition.

Selon un mode de réalisation préféré, le phyllosilicate naturel modifié comprend un phyllosilicate choisi dans le groupe constitué par les vermiculites et les smectites. De préférence, le phyllosilicate peut être choisi dans le groupe constitué par les montmorillonites, bentonites, nontronites, beidellites, volkonskoites, hectorites, saponites, sauconites, sobockites, stevensites, svinfordites, de préférence ce sont des phyllosilicates de sodium, de potassium, de calcium, ou de leurs mélanges. De manière plus préférée, le phyllosilicate est choisi dans le groupe constitué par l'hectorite, la montmorillonite, la bentonite ou leurs mélanges.

Selon un mode de réalisation préféré, le phyllosilicate naturel modifié comprend un composé organique choisi dans le groupe constitué par la gomme xanthane, la gomme guar, la gomme de Tara ou de Caroube, la gomme d'acacia, le carraghénane, l'alginate, le chitosane, la pectine, l'acide citrique, l'acide tartrique, l'acide oxalique, l'acide succinique, l'acide malique, l'acide acétique, l'acide lactique, l'acide propionique, l'acide salicylique, les glycosaminoglycanes. Avantageusement, le phyllosilicate naturel modifié comprend une bentonite modifiée par la gomme de xanthane et l'acide citrique.

Selon un autre mode de réalisation, le phyllosilicate naturel modifié comprend un composé organique choisi parmi tout composé organique connu dans l'art des émulsions de Pickering, tels que ceux divulgués dans la demande de brevet français n° FR 2 976 503.

Avantageusement, le phyllosilicate modifié est présent en une quantité allant de 2% à 20%, de préférence 2,5% à 10% et de manière préférée 3,5% à 8% en poids par rapport au poids total de la composition.

Selon un mode de réalisation préféré, la phase huileuse vegétale de l'émulsion de Pickering comprend au moins une huile végétale choisie dans le groupe formé par l'huile de tournesol, l'huile de colza, l'huile d'olive, l'huile de cameline, l'huile d'arachide, l'huile de coco, l'huile de pépin de raisin, l'huile de ricin, l'huile d'argan, l'huile de Djansang, l'huile de dattier du désert, l'huile de nigelle, l'huile de figue de barbarie, l'huile de macadamia, l'huile de soja, l'huile de palme et de palmier, l'huile de Tamanu, l'huile de sésame, l'huile de lin, l'huile de noix, l'huile de noisette, l'huile de baobab, l'huile de fruit de la passion, l'huile de noix du Brésil, l'huile d'hibiscus, l'huile de pépin de courge, l'huile de Luffa, l'huile de Carapa, l'huile d'Onagre, l'huile de bourrache, huile d'avocat, l'huile d'amande, l'huile d'argousier, l'huile de noyau d'abricot, l'huile de noyau de cerise, l'huile de pépin de pomme, l'huile de grenade, l'huile de Jojoba, l'huile de rose musquée, l'huile de prune, le beurre de karité, le beurre de cacao, le beurre de Kokum, le beurre de mangue, le beurre de moabi, le beurre de Karanja, le beurre de Tucuma, le beurre de Cupuaçu, le beurre de Buriti, le beurre de Murumuru, le beurre de Kombo, le beurre de Kpangnan, les triglycérides d'acides caprylique/caprique.

Avantageusement la composition utilisée dans la présente invention ne comprend pas d'extrait d'huile de chanvre.

Avantageusement, la phase huileuse de l'émulsion de Pickering est présente en une quantité allant de 5% à 40%, de préférence 10% à 30% et de manière préférée 15% à 25% en poids par rapport au poids total de la composition.

**Composition**

La composition utilisable selon l'invention est de préférence une composition vaporisable (« sprayable » en langue anglaise) en fines gouttelettes.

De préférence, la composition utilisable selon l'invention comprend de 43 à 93 % en poids d'eau par rapport au poids total de la composition.

Avantageusement la composition selon la présente invention comprend, dans un milieu acceptable, au moins un agent cosmétique choisi parmi les agents humectants, les épaississants, les agents de texture, les émulsifiants, les agents dispersants, les agents moussants, les émolients, les conservateurs, les colorants, les extraits de plantes, les fibres végétales, les minéraux, les agents correcteurs de pH, les principes actifs et les parfums.

Avantageusement la composition selon la présente invention comprend de 0,001 à 20 % en poids d'au moins un agent cosmétique par rapport au poids total de la composition.

Bien entendu, l'homme du métier veillera à choisir ces agents cosmétiques de manière à ne pas altérer les propriétés de la composition utilisable selon l'invention, notamment de manière à ne pas altérer les propriétés conférant à cette composition son caractère vaporisable.

La composition selon l'invention peut constituer une composition de massage, de soin de la peau, et notamment une crème de nettoyage, de protection, de traitement ou de soin pour le visage, pour les mains, pour les pieds, ou pour le corps, en particulier la composition peut être une crème de jour, crème de nuit, crème démaquillante, crème de fond de teint, crème antisolaire ; un fond de teint fluide, un lait de démaquillage, un lait corporel de protection ou de soin, un lait anti-solaire; une lotion, gel ou mousse pour le soin de la peau, comme une lotion micellaire de nettoyage.

De manière avantageuse, le procédé de préparation de la composition selon l'invention comprend au moins les étapes suivantes dans cet ordre :

- préparer la phase aqueuse ;
- ajouter le phyllosilicate organomodifié à la phase aqueuse et mélanger ;
- ajouter la phase huileuse végétale au mélange obtenu et obtenir une émulsion de Pickering H/E.

**Utilisations**

Avantageusement, l'utilisation selon l'invention est telle que la composition est appliquée par pulvérisation.

Les exemples qui suivent visent à illustrer l'invention sans en limiter la portée.

**Exemples**

**A-Compositions cosmétiques**

Dans le tableau 1 sont répertoriés les produits utilisés pour préparer la composition **A** utilisable selon l'invention.

[Tableau 1]

| Phase | Nom commercial | % | INCI |
|---|---|---|---|
| A | Eau | Qsp | Aqua |
| A | Frametime CXG commercialisé par Ephyla | 2,50 | Bentonite & xanthan gum & Sodium stearoyl glutamate & citric acid |
| B | Huile de tournesol raffinée commercialisée par CAUVIN | 8,00 | Helianthus annuus seed oil |
| C | Georgard Ultra commercialisé par Lonza | 1,00 | Gluconolactone & Sodium benzoate & Calcium gluconate |
| C | Benzoate de Sodium | 0,30 | Sodium benzoate |
| Total | | 100 | |

Les constituants de la phase A ont été mélangés à 20°C avec un mixeur mécanique à couteaux ou un rotor stator à une vitesse de 4000 rpm durant 10 minutes de sorte à mettre en œuvre un cisaillement et une dispersion suffisante pour obtenir l'homogénéisation de la phase.

La phase B a ensuite été incorporée, toujours à 20°C, sur le même modèle de cisaillement progressivement durant 10 minutes.

L'émulsion de pickering a ainsi été réalisée. La température du mélange, sous cisaillement, a ensuite été augmentée pour atteindre 50°C de sorte à pouvoir solubiliser le conservateur.

La phase C (conservateurs) a ensuite été introduite à cette température de 50°C sous une agitation plus modérée de 2000 rpm.

Le produit est refroidi à température ambiante.

Enfin, le pH a été ajusté à 4,9.

La galénique de la composition obtenue comprend des fines gouttelettes d'huile enrobées de plaquettes de bentonite. Ces plaquettes minérales sont stables à l'interface huile/eau, elles sont issues de l'exfoliation de la bentonite (Frametime CXG) sous l'action du cisaillement mécanique et forment des microcapsules d'huile. La dispersion des microcapsules est stable lorsque le produit est au repos même si sa viscosité est faible.

La taille des microcapsules est de l'ordre de 5 à 15 $10^{-6}$ m de diamètre, ce qui permet à cette galénique de passer à travers les buses de vaporisation de type standard en cosmétique.

Cette composition a ainsi été vaporisée sur la peau saine, elle peut alors être massée délicatement pour parfaire l'étalement sur la peau ou préférentiellement, elle peut être laissée à l'état de brume fine à la surface de la peau.

Dans le tableau 2 sont répertoriés les produits utilisés pour préparer la composition **B** utilisable selon l'invention.

[Tableau 2]

| Phase | Nom commercial | % | INCI |
|---|---|---|---|
| A | Eau | 73,70 | Aqua |
| B | Georgard Ultra commercialisé par Lonza | 1,00 | Gluconolactone & Sodium benzoate & Calcium gluconate |
| B | Benzoate de Sodium | 0,30 | Sodium benzoate |
| C | Frametime CX commercialisé par Ephyla | 4,50 | Bentonite & xanthan gum & citric acid |
| C | Gomme de xanthane FF commercialisée par Jungbunzlauer | 0,50 | xanthan gum |
| D | Trigycéride d'acides caprique /caprylique | 20,00 | Caprylic/capric triglyceride |
| Total | | 100 | |

Préparation de la composition **B** :

Les phases A et B ont été mélangées pour former une phase aqueuse puis la phase C a été ajoutée et le tout a été mélangé. Enfin la phase huileuse D a été ajoutée et le tout a été mélangé. Une fois l'émulsion de Pickering obtenue, le pH a été ajusté à 5,00.

La galénique de la composition obtenue comprend des fines gouttelettes d'huile enrobées de plaquettes de bentonite. Ces plaquettes minérales sont stables à l'interface huile/eau, elles sont issues de l'exfoliation de la bentonite (Frametime CXG) sous l'action du cisaillement mécanique et forment des microcapsules d'huile. La dispersion des microcapsules est stable

lorsque le produit est au repos, sa viscosité correspond à celle d'une crème qui tient dans un pot de crème standard.

Cette composition a été étalée manuellement sur la peau saine d'un avant bras. La crème étalée procure un effet caresse durable, réconfortant et apporte une sensation de bien-être.

Ce type de galénique « microencapsulée » permet de déposer ou brumiser une couche de microcapsules contenant des molécules lipidiques de soin de la peau. En séchant, les microcapsules libèrent progressivement les molécules lipidiques de soin (la phase huileuse), ce qui permet d'apporter un effet hydratant et des substances nourrissantes avec une grande rémanence, améliorer l'hydratation de la peau et maintenir ou restaurer l'éclat du teint de la peau au fur et à mesure de la libération de microcapsules.

Selon la nature de la peau les microcapsules libèrent leur contenu plus ou moins vite : plus la peau est sèche plus les microcapsules libèrent leur contenu rapidement ; sur une surface dite mixte (avec une zone grasse et une zone sèche), les microcapsules libèrent leur contenu rapidement sur la zone sèche, alors que sur la zone grasse les microcapsules vont rester intactes plus durablement. Ainsi la galénique participe à équilibrer la peau en surface en paliant spécifiquement à la sécheresse cutanée des zones les plus sèches et en n'ajoutant pas de corps gras sur les zones grasses.

Cet effet équilibrant permet à la peau de limiter ses contrastes et confère donc un aspect plus uniforme et plus lisse. Cet effet de « gommage » des imperfections (accentuées par les contrastes qui creusent le relief cutané) confère à la peau une apparence plus jeune et déstressée. Le confort pour l'utilisateur est corrélé à libération progressive des microcapsules de soin au fur et à mesure des besoins de la peau.

**B- Mesure de l'effet neurocosmétique sur explants de peau humaine par mesure de l'augmentation de la production de sérotonine**

Les cellules de Merkel de la peau, libérent des neurotransmetteurs et en particulier de la serotonine de sorte à transmettre au cerveau l'information d'un toucher « caresse » *via* la fibrille nerveuse sous-jacente. C'est ainsi que les mecanorécepteurs (cellules de Merkel ou disques de Merkel) assurent le sens

du toucher notamment sur les sensations positives ; il s'agit de transcrire un effet mécanique, à savoir le toucher « caresse », en message chimique, à savoir la sérotonine, un neuromédiateur que le cerveau assimile au bien-être

Cette étude est basée sur la capacité de la composition conforme à l'invention **A** à augmenter la production de sérotonine.

L'effet recherché est un effet stimulateur sur la production de sérotonine qui se traduit par un effet de réconfort, une sensation de bien-être, un effet caresse sur l'épiderme qui est en contact avec le milieu extérieur.

*Compositions testées*

La composition A est utilisée comme composition conforme à l'invention.

A titre de comparaison, ont été utilisés :

- du sérum physiologique c'est-à-dire une composition de NaCl à 0.9%, à titre de témoin basal (TB) et
- de l'isothiocyanate de1-naphtyle à la concentration de 0,25% (V/V) à titre de témoin positif (TP).

*Principe de l'étude*

Pour cette étude des explants cutanés humains frais normaux avec cellules de Merkel sont incubés pendant 20 heures à 32°C pour acclimatation.

Puis les échantillons d'explants à tester -en triplicat- sont traités par pulvérisation de la composition A utilisée conformément à l'invention ou par pulvérisation des compositions témoins TB et TP et replacés en incubation à 32°C.

Au bout de 3 heures, une première série d'échantilllons d'explants traités a été récupérée et il a été procédé à la dissection et à la mise en préparation des explants traités : placement dans l'azote liquide pendant 3 minutes, puis dans 10 mL de PBS 1X glacé puis dans un bain d'ultrasons pendant 45 minutes de sorte à obtenir un lysat cellulaire.

Après ce traitement réalisé pour chaque implant, 2mL de lysat cellulaire de chaque échantilllon d'explant de cette première série ont été prélevés et 1% d'agent stabilisant y a été ajouté pour les conserver à -20°C jusqu'à la réalisation du dosage Elisa.

Les explants traités pendant 3 heures au moyen des compositions A, TB et TP sont respectivement nommés A3, TB3 et TP3.

Au bout de 8 heures, une deuxiéme série d'échantilllons d'explants traités a été récupérée et traitée comme ci-dessus. Puis 2mL de lysat cellulaire de chaque échantilllon d'explant de cette deuxième série ont été prélevés et 1% d'agent stabilisant y a été ajouté pour les conserver à -20°C jusqu'à la réalisation du dosage Elisa.

Les explants traités pendant 8 heures aux compositions A, TB et TP sont respectivement nommés A8, TB8 et TP8.

Au moment de la réalisation du dosage Elisa, les échantilllons d'explants traités ont été soumis à un nouveau bain à ultrasons pendant 35 minutes puis, après homogénéisation, les surnageants sont récupérés en vue de la réalisation du test Elisa - sérotonine.

*Système d'essai Elisa - sérotonine*

Les solutions de standard et de contrôle ont été préparées conformément aux indications du kit Elisa - sérotonine (référence commerciale du kit : Serotonin Research ELISA ® commercialisé par Immusmol).

Le kit fournit un test pratique pour le dosage de la concentration en sérotonine des milieux comprenant les surnageants d'explants cutanés préparés permettant ainsi d'évaluer l'effet modulateur de la composition topique à tester.

Ce kit met en œuvre une méthode d'immunodosage enzymatique ultrasensible pour la détermination quantitative de la sérotonine. La sérotonine est acylée, puis détectée par les antigènes qui sont liés à la phase solide de la plaque de microtitrage. Les normes, contrôles et échantillons acylés et les analytes liés à la phase solide sont en concurrence pour un nombre fixe de sites de liaison d'anticorps. Une fois le système en équilibre, l'antigène libre et les complexes antigènes libres-anticorps sont éliminés par lavage. L'anticorps lié à la phase solide est détecté par un conjugué IgG-peroxydase anti-lapin en utilisant TMB ((3,3',5,5'-Tetramethylbenzidine) comme substrat. Il s'agit donc d'un test colorimétrique dont la réaction colorée est révélée à 450 nm. La quantification des échantillons inconnus est obtenue en comparant leur absorbance avec une courbe standard préparée avec des concentrations standard connues.

*Résultats*

Gamme étalon

Pour cette étude une gamme d'étalonnage en sérotonine allant de 0,0 à 2,5 $10^{-9}$ g/mL (ng/mL) a été réalisée. Les absorbances de la gamme étalon en sérotonine à 450 nm sont présentés dans le tableau 3.

[Tableau 3]

| $DO_{450nm}$ | | | | | | |
|---|---|---|---|---|---|---|
| | Blanc | **Standards** = Gamme sérotonine ($10^{-9}$ g/mL) | | | | |
| | S0 | S1 = 0,000 | S2 = 0,015 | S3 = 0,050 | S4 = 0,150 | S5 = 0,250 |
| Replicat 1 | 12,545 | 11,633 | 11,095 | 9,839 | 6,903 | 4,309 |
| Replicat 2 | 12,555 | 11,698 | 10,982 | 9,679 | 7,263 | 3,895 |
| Replicat 3 | 13,053 | 12,929 | 9,643 | 9,074 | 6,760 | 4,182 |
| Moyenne | 12,718 | 12,087 | 10,573 | 9,531 | 6,975 | 4,129 |
| *Ecart type* | *0,290* | *0,730* | *0,807* | *0,403* | *0,259* | *0,212* |

L'équation de la courbe d'étalonnage est :

$$y = 28,673x^2 - 25,137x + 12,393$$

le coefficient de détermination est : $R^2$ = 0,9719

Mesure des échantillons

Les résultats d'absorbance (DO-densité optique) à 450 nm avec écarts types de la composition conforme A et des témoins TB et TP à 3 heures et 8 ainsi que le pourcentage (%) d'augmentation par rapport au témoin basal heures sont présentés respectivement dans le tableau 4, ci-dessous

[Tableau 4]

| DO 450 nm de la composition A et des témoins et ecart type | | | | | | |
|---|---|---|---|---|---|---|
| | TB3 | TP3 | A3 | TB8 | TP8 | A8 |
| DO 450nm | 0,204 | 0,340 | 0,246 | 0,203 | 0,254 | 0,246 |
| Ecart type | 0,020 | 0,031 | 0,028 | 0,026 | 0,060 | 0,025 |
| % d'augmen tation par rapport au témoin basal | 0 | 66,67 | 20,58 | 0 | 25,12 | 21,18 |

Dans les conditions de l'étude, la cinétique de réponse positive par rapport au temps de mise en contact avec les explants de peau montre que le pic de stimulation attendu avec le témoin positif (l'isothiocyanate de 1-naphtyle) s'observe après 3 heures de contact avec + 66,67% de sérotonine produite par rapport au témoin explant de peau sans produit à tester. Cette réponse positive au témoin positif valide l'expérience. Après 8 heures de contact l'effet positif du témoin positif diminue très significativement, l'effet est décroissant entre 3 heures de contact et 8 heures de contact.

Avec la composition A selon l'invention, l'augmentation de la production de serotonine, par rapport au témoin basal TB3 est de 20,58% (A3) au bout de 3 heures (A3), et reste identique au bout de 8 heures (21,18% pour A8). L'effet positif de la composition selon l'invention est donc stable dans le temps ce qui témoigne d'une rémanence sur cet effet positif.

Conclusion :

La composition selon l'invention **A** appliquée sur les explants exposés stimule la production de sérotonine de plus de 20% par rapport au témoin basal après 3 heures de mise en contact (A3) avec l'explant, cette production se maintient à + 21% par rapport au témoin basal après 8 heures de mise en contact (A8).

Il est rappelé que dans la peau les cellules de Merkel sont les mécano-récepteurs en première ligne du sens du toucher, ainsi que les principales cellules capables de produire de la sérotonine en quantité significative. La sérotonine est un neuro-médiateur de la sensation de caresse qui par la fibrille nerveuse sous-jacente aux cellules de Merkel permet d'informer le cerveau d'une sensation agréable est positive.

Ainsi, la composition selon l'invention A par augmentation de la production du neuro-médiateur sérotonine informe le cerveau d'une modulation positive de type caresse ou effet sensoriel positif.

La composition selon l'invention A par augmentation de la production du neuro-médiateur sérotonine a également un effet anti-âge.

## BIBLIOGRAPHIE

*1.* Effects on tactile transmission by serotonin transporter inhibitors at Merkel discs of mouse whisker hair follicles, Chang and G Gu, Molecular Pain vol. 16 : 1-9, 20 may 2020;

*2.* Neurocosmetics in Skincare -The Fascinating World of Skin-Brain Connection: A Review to Explore Ingredients, Commercial Products for Skin Aging, and Cosmetic Regulation. Rizzi, V.; Gubitosa, J.; Fini, P.; Cosma, P. Cosmetics 2021, 8, 66.

*3. 윤담지 . The effects of skin-derived oxytocin or serotonin on brain function and skin aging in mice. 2024. Thèse de doctorat.서울대학교 대학원 ( Citation de la publication selon la norme ISO 690)*

## Revendications

1. Utilisation cosmétique, non thérapeutique, par voie topique d'une composition en tant qu'agent neurocosmétique, ladite composition comprenant une émulsion de Pickering H/E contenant une phase aqueuse, une phase huileuse végétale et un phyllosilicate organomodifié.

2. Utilisation selon la revendication 1 dans laquelle le phyllosilicate organomodifié est présent en une quantité allant de 2% à 20%, de préférence 2,5% à 10% et de manière préférée 3,5% à 8% en poids par rapport au poids total de la composition.

3. Utilisation selon l'une quelconque des revendications 1 ou 2 dans laquelle la phase aqueuse de l'émulsion de Pickering est présente en une quantité allant de 43% à 93%, de préférence 55% à 70% et de manière préférée 58% à 65% en poids par rapport au poids total de la composition.

4. Utilisation selon l'une quelconque des revendications 1 à 3 dans laquelle la phase huileuse vegétale de l'émulsion de Pickering comprend au moins une huile végétale choisie dans le groupe formé par l'huile de tournesol, l'huile de colza, l'huile d'olive, l'huile de cameline, l'huile d'arachide, l'huile de coco, l'huile de pépin de raisin, l'huile de ricin, l'huile d'argan, l'huile de Djansang, l'huile de dattier du désert, l'huile de nigelle, l'huile de figue de barbarie, l'huile de macadamia, l'huile de soja, l'huile de palme et de palmier, l'huile de Tamanu, l'huile de sésame, l'huile de lin, l'huile de noix, l'huile de noisette, l'huile de baobab, l'huile de fruit de la passion, l'huile de noix du Brésil, l'huile d'hibiscus, l'huile de pépin de courge, l'huile de Luffa, l'huile de Carapa, l'huile d'Onagre, l'huile de bourrache, huile d'avocat, l'huile d'amande, l'huile d'argousier, l'huile de noyau d'abricot, l'huile de noyau de cerise, l'huile de pépin de pomme, l'huile de grenade, l'huile de Jojoba, l'huile de rose musquée, l'huile de prune, le beurre de karité, le beurre de cacao, le beurre de Kokum, le beurre de mangue, le beurre de moabi, le beurre de Karanja, le beurre de Tucuma, le beurre de Cupuaçu, le beurre de Buriti, le beurre de Murumuru, le beurre de Kombo, le beurre de Kpangnan, les triglycérides d'acides caprylique/ caprique.

5. Utilisation selon l'une quelconque des revendications 1 à 4 dans laquelle la phase huileuse de l'émulsion de Pickering est présente en une quantité allant de 5% à 40%, de préférence 10% à 30% et de manière préférée 15% à 25% en poids par rapport au poids total de la composition.

6. Utilisation selon l'une quelconque des revendications 1 à 5 comprenant, dans un milieu acceptable, au moins un agent cosmétique choisi parmi les agents humectants, les épaississants, les agents de texture, les émulsifiants, les agents dispersants, les agents moussants, les émolients, les conservateurs, les colorants, les extraits de plantes, les fibres végétales, les minéraux, les agents correcteurs de pH, les principes actifs et les parfums.

7. Utilisation selon l'une quelconque des revendications 1 à 6 dans laquelle la composition est appliquée par pulvérisation.

8. Utilisation selon l'une quelconque des revendications 1 à 7 pour apporter à la peau un effet réconfortant, une sensation de bien-être.

9. Utilisation selon la revendication 1 à 7 pour améliorer l'hydratation de la peau et/ou améliorer l'éclat de la peau.

10. Utilisation selon la revendication 1 à 7 pour conférer à la peau un aspect plus lisse, plus clair et/ou plus uniforme.

11. Utilisation selon la revendication 1 à 7 pour son effet anti-âge, antiride et/ou anti-tâche.

12. Procédé de traitement cosmétique, non thérapeutique de la peau, comprenant au moins une étape d'application sur la peau d'une composition neurocosmétique comprenant une émulsion de Pickering H/E contenant une phase aqueuse, une phase huileuse végétale et un phyllosilicate organomodifié.[ ]

Europäisches
Patentamt

European
Patent Office

Office européen
des brevets

## RAPPORT DE RECHERCHE EUROPEENNE

Numéro de la demande

EP 25 21 8356

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2021/085577 A1 (BOURGETEAU VINCENT [FR] ET AL) 25 mars 2021 (2021-03-25) * alinéas [[0002]], [[0012]], [[0059]] - [[0080]], [[0110]] - [[0115]]; revendications 23-25; tableaux 6,7 * ----- | 1-12 | INV. A61K8/06 A61K8/26 A61K8/92 A61Q19/00 A61Q19/08 |
| A | Anon: "Function: cold emulsifier for", , 28 juillet 2015 (2015-07-28), XP055319665, Extrait de l'Internet: URL:http://www.ephyla.fr/wp-content/uploads/premium/Ephyla_FT Frametime CXG_240512-ENG.pdf [extrait le 2016-11-15] * le document en entier * ----- | 1-7,12 | |
| A | Anonymous: "FRAMETIME CX ¦ Ephyla", , 29 juillet 2015 (2015-07-29), XP055319655, Extrait de l'Internet: URL:https://web.archive.org/web/2015072900 2709/http://www.ephyla.fr/en/product/frame time-cx/ [extrait le 2016-11-15] * le document en entier * ----- | 1-7,12 | |
| A | Anon: "Function", , 29 juillet 2015 (2015-07-29), XP055319660, Extrait de l'Internet: URL:http://www.ephyla.fr/wp-content/uploads/premium/Ephyla_FT Frametime CX_240512-ENG.pdf [extrait le 2016-11-15] * le document en entier * ----- | 1-7,12 | |

DOMAINES TECHNIQUES
RECHERCHES (IPC)

A61K
A61Q

Le présent rapport a été établi pour toutes les revendications

| Lieu de la recherche | Date d'achèvement de la recherche | Examinateur |
|---|---|---|
| Munich | 25 mars 2026 | Yon, Jean-Michel |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons
...........................................................................
& : membre de la même famille, document correspondant

EPO FORM 1503 03.82 (P04C02)

## EP 4 751 703 A1

**ANNEXE AU RAPPORT DE RECHERCHE EUROPEENNE**
**RELATIF A LA DEMANDE DE BREVET EUROPEEN NO.**

EP 25 21 8356

25-03-2026

| Document brevet cité au rapport de recherche | Date de publication | Membre(s) de la famille de brevet(s) | Date de publication |
|---|---|---|---|
| US 2021085577 A1 | 25-03-2021 | CA 3002130 A1 | 20-04-2017 |
| | | EP 3362035 A1 | 22-08-2018 |
| | | US 2021085577 A1 | 25-03-2021 |
| | | WO 2017064686 A1 | 20-04-2017 |

EPO FORM P0460

## RÉFÉRENCES CITÉES DANS LA DESCRIPTION

**Documents brevets cités dans la description**

- FR 2976503

**Littérature non-brevet citée dans la description**

- **CHANG** ; **G GU**. Effects on tactile transmission by serotonin transporter inhibitors at Merkel discs of mouse whisker hair follicles. *Molecular Pain*, 20 May 2020, vol. 16, 1-9

- **RIZZI, V.** ; **GUBITOSA, J.** ; **FINI, P.** ; **COSMA, P**. Neurocosmetics in Skincare -The Fascinating Worid of Skin-Brain Connection: A Review to Explore Ingredients, Commercial Products for Skin Aging, and Cosmetic Regulation. *Cosmetics*, 2021, vol. 8, 66